# EUROPEAN PATENT SPECIFICATION

(11) **EP 3 897 730 B1**
(45) Date of publication and mention of the grant of the patent: **09.12.2026**
(21) Application number: 19836896.1
(22) Date of filing: 17.12.2019
(51) Int. Cl.: A61K 47/38, A61K 9/48, C08L 1/28

(54) **A SUSTAINED RELEASE COMPOSITION COMPRISING A METHYLCELLULOSE**
ZUSAMMENSETZUNG MIT EINER METHYLCELLULOSE MIT VERZÖGERTER FREISETZUNG
COMPOSITION À LIBÉRATION PROLONGÉE COMPRENANT UNE MÉTHYLCELLULOSE

(30) Priority: 18.12.2018 EP 18213453
(43) Date of publication of application: 27.10.2021
(73) Proprietor: DDP Specialty Electronic Materials US, Inc., Collegeville, PA 19426 (US)
(72) Inventor: PETERMANN, Oliver, 29699 Bomlitz (DE)
(74) Representative: f & e patent
(86) International application number: PCT/US2019/066712
(87) International publication number: WO 2020/131784

(56) References cited:
- WO-A1-2013/059064
- US-A- 4 717 713
- US-A1- 2009 304 789
- MITCHELL K ET AL: "The influence of substitution type on the performance of methylcellulose and hydroxypropylmethycellulose in gels and matrices", INTERNATIONAL JOURNAL OF PHARMACEUTICS, ELSEVIER, NL, vol. 100, no. 1-3, 8 November 1993 (1993-11-08), pages 143 - 154, XP025554113, ISSN: 0378-5173, [retrieved on 19931108], DOI: 10.1016/0378-5173(93)90085-T
- KUNIHIKO ITOH ET AL: "In situ gelling formulation based on methylcellulose/pectin system for oral-sustained drug delivery to dysphagic patients", DRUG DEVELOPMENT AND INDUSTRIAL PHARMACY, vol. 37, no. 7, 19 July 2011 (2011-07-19), US, pages 790 - 797, XP055565253, ISSN: 0363-9045, DOI: 10.3109/03639045.2010.541465

## Description

### FIELD

The present invention relates to novel sustained release compositions comprising a physiologically active ingredient and a methylcellulose.

### INTRODUCTION

Sustained release dosage forms have found wide application in a variety of technology areas such as in personal care and agricultural applications, water treatment and in particular pharmaceutical applications. Sustained release dosage forms are designed to release a finite quantity of an active ingredient into an aqueous environment over an extended period of time. Sustained release pharmaceutical dosage forms are desirable because they provide a method of delivering a long-lasting dose in a single application without overdosing. Known sustained release pharmaceutical dosage forms contain a drug or a vitamin whose release is controlled by a polymeric matrix which, for instance, may comprise one or more water-soluble cellulose ethers. Water-soluble cellulose ethers hydrate on the surface of a tablet to form a gel layer. A fast formation of the gel layer is important to prevent wetting of the interior and disintegration of the tablet core. Once the gel layer is formed, it controls the penetration of additional water into the tablet. As the outer layer fully hydrates and dissolves, an inner layer must replace it and be sufficiently cohesive and continuous to retard the influx of water and control drug diffusion.

A commonly used cellulose ether for providing sustained release of an active ingredient from an oral dosage form is hydroxypropyl methylcellulose (HPMC). For instance, US 4,734,285 discloses that the release of an active ingredient can be prolonged by employing a fine particle sized HPMC as an excipient in a solid tablet. HPMC is used in commercial oral pharmaceutical formulations as a component of a polymeric matrix providing sustained release of a drug usually at a concentration of 30% to 60% by weight of the oral dosage form.

K Mitchell et al., 1993, International Journal of Pharmaceutics 100(1-3): 143-154, studies the characteristics of matrices containing various hydroxypropylmethylcellulose or methylcellulose grades, via the dissolution of a model drug from matrices prepared by direct compression.

It is a well-known problem in the pharmaceutical art that some patients, especially children or the elderly, or patients with dysphagia, find it difficult to swallow conventional oral dosage forms such as capsules or tablets. In particular, this is the case if the drug administered in the dosage form is a highly dosed drug which, when the drug is formulated with pharmaceutical excipients in the typical amounts included in commercial dosage forms, either makes each dosage form very large or requires the dose to be divided among two or more dosage forms that have to be swallowed at the same time.

It would therefore be desirable to develop an oral dosage form where a drug is formulated with a reduced amount of excipient(s) to permit a reduction in the overall size of the dosage form and improve the swallowability without compromising the sustained release properties thereof.

### SUMMARY OF THE INVENTION

It has surprisingly been found that when a methylcellulose with a gelling temperature which is higher than body temperature is used as an excipient in admixture with a physiologically active ingredient, it is capable of forming a stable hydrogel at a temperature of 37°C and provide sustained release of the active ingredient even when it is used at much lower concentrations that the concentrations of HPMC used in commercial formulations.

Accordingly, the present invention relates to a sustained release composition for oral administration comprising a physiologically active ingredient mixed with a methylcellulose, wherein
the methylcellulose has anhydroglucose units joined by 1-4 linkages and wherein hydroxy groups of anhydroglucose units are substituted with methyl groups such that the s23/s26 is between 0.27 and 0.33,
wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups, and
wherein the concentration of methylcellulose is from 0.1% to 10% by dry weight of the active ingredient.

While EP 1171471 B1 discloses a methylcellulose with enhanced gel strength, which has a level of methoxy substitution of 21-42% by weight and a gelling temperature of 31-54°C, and its use in many different applications, including as an excipient in sustained release and timed release tablets, it is not suggested that the methylcellulose can be used as a polymeric matrix material in a very low concentration in a solid dosage form compared to the concentration of the active ingredient while retaining its sustained release properties.

The use of methylcellulose as a controlled release matrix material is disclosed in K.S. Aithal et al., Indian J. Dent. Res. 1, Apr-Sep 1990, 174-181. Chewable tablets containing 2% by weight of NaF, 30% by weight of methylcellulose, 63% by weight of lactose and 5% by weight of starch powder. The authors report that about 80% of the NaF is released within 20 minutes from this formulation, while the release of NaF from tablets containing either more or less methylcellulose is about 15 minutes. There is no indication that methylcellulose may be used as an excipient providing sustained release over a period of several hours.

### BRIEF DESCRIPTION OF THE DRAWINGS

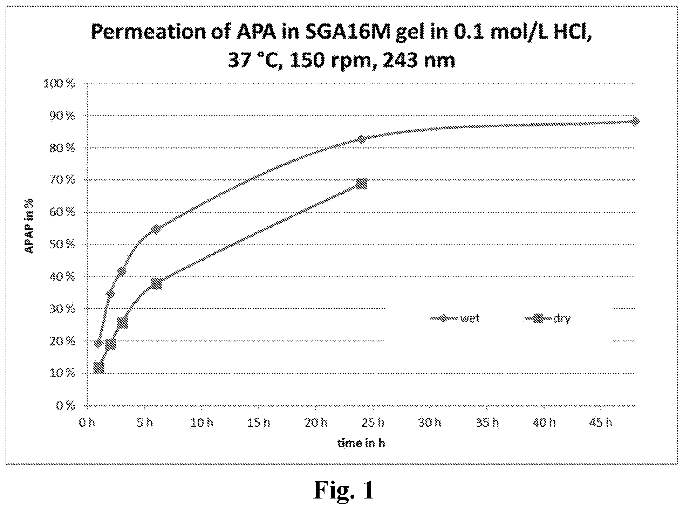

Fig. 1 is a graph showing the release over time of acetaminophen (APAP) from a composition of the invention containing a 2% solution of SGA16M methylcellulose when a gelatin capsule containing the composition is immersed in 900 ml of 0.1 N HCl pH 1.1. Release from a wet capsule is shown as -◆-, and release from a capsule dried overnight at room temperature is shown as -■-.

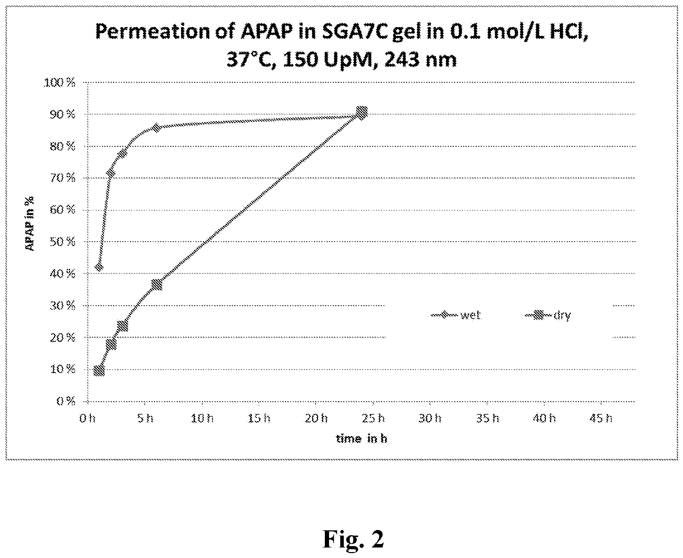

Fig. 2 is a graph showing the release over time of acetaminophen (APAP) from a composition of the invention containing a 2% solution of SGA7C methylcellulose when a gelatin capsule containing the composition is immersed in 900 ml of 0.1 N HCl pH 1.1. Release from a wet capsule is shown as **-◆-**, and release from a capsule dried overnight at room temperature is shown as -■-.

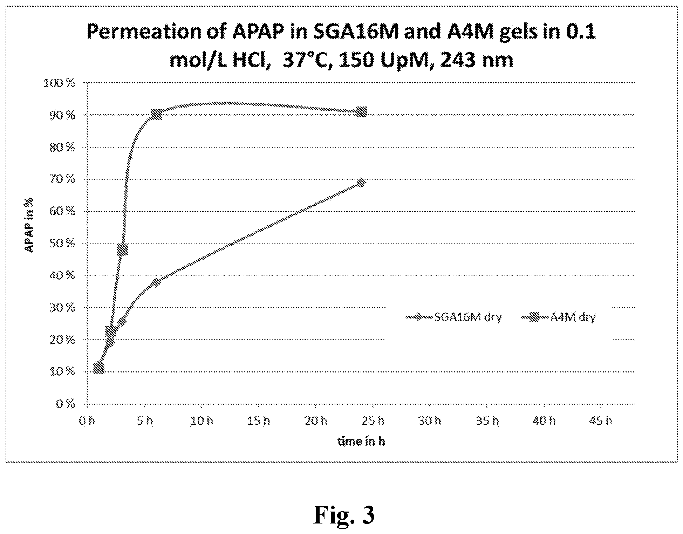

Fig. 3 is a graph showing the release over time of acetaminophen (APAP) from a gelatin capsule containing a composition of the invention containing a 2% solution of SGA16M methylcellulose dried overnight at room temperature and then immersed in 900 ml of 0.1 N HCl pH 1.1 (shown as **-◆-**), and release of APAP from a gelatin capsule containing a composition of the invention containing a 2% solution of A4M methylcellulose dried overnight at room temperature and then immersed in 900 ml of 0.1 N HCl pH 1.1 (shown as **-■-**).

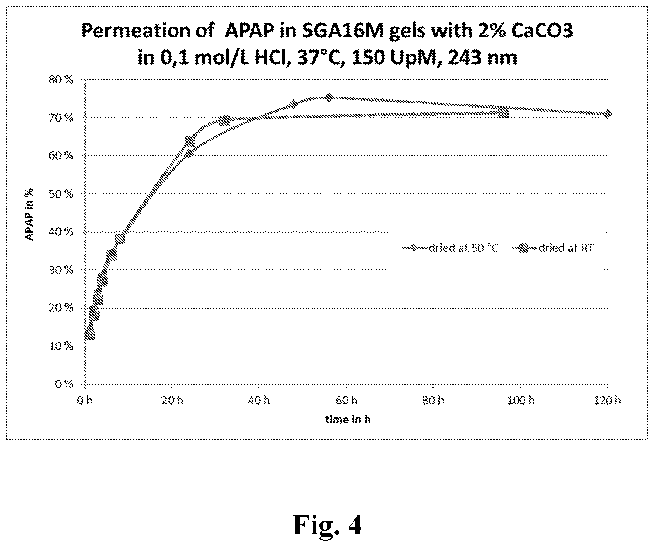

Fig. 4 is a graph showing the release over time of acetaminophen (APAP) from a gelatin capsule containing a composition of the invention containing a 2% solution of SGA16M methylcellulose and 2% CaCO₃, dried overnight at 50°C (shown as **-◆-**) and at room temperature for 2 days (shown as **-■-**), and immersed in 900 ml of 0.1 N HCl pH 1.1 at 37oC and 150 rpm.

### DESCRIPTION OF EMBODIMENTS

In the present invention, methylcellulose is an essential component of the composition to form a hydrogel in an aqueous environment such as the stomach and provide sustained release of the active ingredient on oral administration of the composition even when the methylcellulose is present in a very low amount relative to the active ingredient. The methylcellulose has anhydroglucose units joined by 1-4 linkages. Each anhydroglucose unit contains hydroxyl groups at the 2, 3, and 6 positions. Partial or complete substitution of these hydroxyls creates cellulose derivatives. For example, treatment of cellulosic fibers with caustic solution, followed by a methylating agent, yields cellulose ethers substituted with one or more methoxy groups. If not further substituted with other alkyls, this cellulose derivative is known as methylcellulose.

The position of the methyl groups on the anhydroglucose units is important for the dissolution temperature and gelling temperature of the methylcellulose and consequently for the capacity of the methylcellulose to provide sustained release of an active ingredient. Despite this general trend, the present inventor has surprisingly found that methylcellulose wherein hydroxy groups of the anhydroglucose units are substituted with methyl groups such that s23/s26 is more than 0.27 can form a stable hydrogel at about 37°C when included in the composition at concentrations that are sufficient to embed particles of the active ingredient. While such concentrations may vary between wide limits, and while generally more sustained release may be obtained at higher concentrations of methylcellulose (e.g. 30-60% by weight), it has surprisingly been found that methylcellulose at low concentrations, i.e. concentrations of 10% or less by dry weight of the active ingredient, may be sufficient to cause the active ingredient to become embedded to an extent providing sustained release of the active ingredient over 24 hours.

The composition of the invention comprises a methylcellulose wherein hydroxy groups of anhydroglucose units are substituted with methyl groups such that s23/s26 is between 0.27 and 0.33 and preferably between 0.27 and 0.30. Methylcelluloses wherein hydroxy groups of anhydroglucose units are substituted with methyl groups such that s23/s26 is about 0.29 are commercially available under the trade name METHOCEL SG or SGA (DuPont). They gel at a relatively low temperature, i.e. at 38-44°C, at a concentration of 2% by weight in water. EP 1171471 B1 discloses the preparation of methylcelluloses which, at a concentration of 1.5% by weight in water, exhibit gelation temperatures of 31-54°C, while most of them exhibit gelation temperatures of 35-45°C. As these methylcelluloses generally exhibit dissolution temperatures of 15-20°C, the present compositions can be prepared at room temperature and do not require cooling during the production process, which simplifies the process and makes it less costly.

In the ratio s23/s26, s23 is the molar fraction of anhydroglucose units wherein only the two hydroxy groups in the 2- and 3-positions of the anhydroglucose unit are substituted with methyl groups and s26 is the molar fraction of anhydroglucose units wherein only the two hydroxy groups in the 2- and 6-positions of the anhydroglucose unit are substituted with methyl groups. For determining the s23, the term "the molar fraction of anhydroglucose units wherein only the two hydroxy groups in the 2- and 3-positions of the anhydroglucose unit are substituted with methyl groups" means that the two hydroxy groups in the 2- and 3-positions are substituted with methyl groups and the 6-positions are unsubstituted hydroxy groups. For determining the s26, the term "the molar fraction of anhydroglucose units wherein only the two hydroxy groups in the 2- and 6-positions of the anhydroglucose unit are substituted with methyl groups" means that the two hydroxy groups in the 2- and 6-positions are substituted with methyl groups and the 3-positions are unsubstituted hydroxy groups.

Formula I below illustrates the numbering of the hydroxy groups in anhydroglucose units.

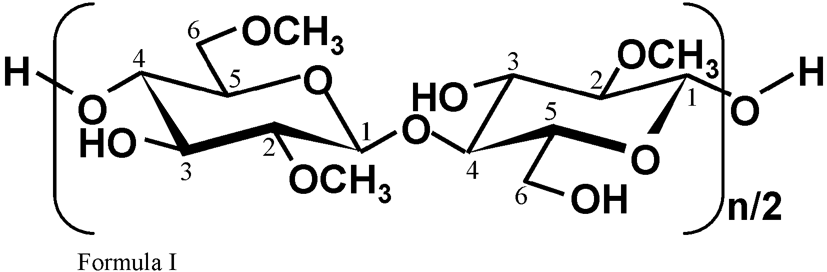

Described are methylcelluloses in which hydroxy groups of anhydroglucose units are substituted with methyl groups such that the s23/s26 of the methylcellulose is 0.8 or less, preferably 0.6 or less, more preferably 0.5 or less (this grade of methylcellulose is termed A methylcellulose in the following). Normally, such as methylcellulose has a high gelling temperature and would not be expected to form a hydrogel at about 37°C, but when a composition is prepared using an amount of liquid diluent below the amount of solids, it has surprisingly been found that a hydrogel can form under the experimental conditions reported in example 3, possibly because the methylcellulose is slowly hydrated in the aqueous environment. An example of such a methylcellulose is Methocel A4M methylcellulose used in example 3 below (available from DuPont). A4M methylcellulose has a DS(methyl) of 1.82 (30% methoxy), an s23/s26 of 0.38-0.42 and a steady-shear-flow viscosity η (5°C, 10 s⁻¹, 2% by weight of methylcellulose) of 4580 mPa.s.

According to the invention s23/s26 of the methylcellulose is between 0.27 and 0.33 and preferably between 0.27 and 0.30. In the following, this methylcellulose is referred to as "SG methylcellulose".

The SG methylcellulose preferably has a DS(methyl) of from 1.55 to 2.25, more preferably from 1.65 to 2.20, and most preferably from 1.70 to 2.10. The degree of the methyl substitution, DS(methyl), also designated as DS(methoxyl), of a methylcellulose is the average number of OH groups substituted with methyl groups per anhydroglucose unit.

The determination of the % methoxyl in methylcellulose is carried out according to the United States Pharmacopeia (USP 34). The values obtained are % methoxyl. These are subsequently converted into degree of substitution (DS) for methyl substituents. Residual amounts of salt have been taken into account in the conversion.

The viscosity of the methylcellulose is generally at least 2.4 mPa•s, preferably at least 3 mPa•s, and most preferably at least 10 mPa•s, when measured as a 2 wt. % aqueous solution at 5 °C at a shear rate of 10 s⁻¹. The viscosity of the methylcellulose is preferably up to 10,000 mPa•s, more preferably up to 5000 mPa•s, and most preferably up to 2000 mPa•s, when measured as indicated above.

In an aqueous environment, the SG methylcellulose is capable of gelling at 37°C at very low concentrations, forming stable hydrogels in an aqueous environment. The term "stable hydrogels", when used in this context is intended to mean hydrogels that retain their shape and are not completely dissolved or significantly eroded after immersion in 0.1 N HCl, pH 1.1, for 4 hours at 37°C. The gelation temperature may for instance be determined as described in EP 1171471 B1.

Examples of SG methylcelluloses are Methocel SGA16M methylcellulose and Methocel SGA7C methylcellulose (both available from DuPont). SGA16M has a DS(methyl)of 1.83 (30% methoxy), and s23/s26 of 0.29 and a steady-shear-flow viscosity η (5°C, 10 s⁻¹, 2% by weight methylcellulose) of 9540 mPa.s. SGA7C has a DS(methyl)of 1.83 (30% methoxy), and s23/s26 of 0.29 and a steady-shear-flow viscosity η (5°C, 10 s⁻¹, 2% by weight methylcellulose) of 1255 mPa.s.

Methylcelluloses may be prepared by the following general method: cellulose pulp is treated with a caustic, for example alkali metal hydroxide. Preferably, about 1.5 to about 3.0 mol NaOH per mol anhydroglucose units in the cellulose is used. Uniform swelling and alkali distribution in the pulp is optionally controlled by mixing and agitation. The rate of addition of aqueous alkaline hydroxide is governed by the ability to cool the reactor during the exothermic alkalization reaction. In one embodiment, an organic solvent such as dimethyl ether is added to the reactor as a diluent and coolant. Likewise, the headspace of the reactor is optionally purged with an inert gas (such as nitrogen) to minimize unwanted reactions with oxygen and molecular weight losses of the methylcellulose. In one embodiment, the temperature is maintained at or below 45°C.

A methylating agent such as methylene chloride is also added by conventional means to the cellulose pulp either before or after or concurrently with the caustic, generally in an amount of 2.0 to 3.5 mol methylating agent per mol anhydroglucose units in the cellulose. Preferably, the methylating agent is added after the caustic. Once the cellulose has been contacted with caustic and methylating agent, the reaction temperature is increased to about 75°C and reacted at this temperature for about half an hour.

In a preferred embodiment, a staged addition is used, i.e. a second amount of caustic is added to the mixture over at least 30 minutes, preferably at least 45 minutes, while maintaining the temperature at 20 to 70°C. Preferably 2 to 4 mol caustic per mol of anhydroglucose units in the cellulose is used. A staged second amount of methylating agent is added to the mixture either before, after or concurrently with the caustic, generally in an amount of 2 to 4.5 mol methylating agent per mol of anhydroglucose units in the cellulose. Preferably, the second amount of methylating agent is added prior to the second amount of caustic.

The methylcellulose is washed to remove salt and other reaction by-products. Any solvent in which salt is soluble may be employed, but water is preferred. The methylcellulose may be washed in the reactor, but is preferably washed in a separate washer located downstream of the reactor. Before or after washing, the methylcellulose may be stripped by exposure to steam to reduce residual organic content. The cellulose ether may subsequently be subjected to a partial depolymerizing process. Partial depolymerizing processes are known in the art and described in e.g. EP 1141029, EP 210917, EP 1423433 and US 4316982. Alternatively, partial depolymerization can be achieved during the production of the cellulose ether, for example by the presence of oxygen or an oxidizing agent. Partial depolymerization results in different properties of the methylcellulose in terms of release rate. Generally, the release rate is higher from a lower molecular weight methylcellulose than from a higher molecular weight methylcellulose as the gel strength of the hydrogel formed in an aqueous environment is lower and the diffusion rate from the hydrogel is consequently higher, cf. the release rates shown for SGA16M methylcellulose and SGA7C methylcellulose in Figs. 1 and 2, respectively, where SGA7C methylcellulose has a lower molecular weight.

The methylcellulose is dried to a reduced moisture and volatile content of preferably 0.5 to 10.0% by weight of water and more preferably 0.8 to 5.0% by weight of water and volatiles based on the weight of methylcellulose. The reduced moisture and volatiles content enables the methylcellulose to be milled into particulate form. The methylcellulose is milled to particulates of a desired size. If desired, drying and milling may be carried out simultaneously.

The SG methylcellulose and A methylcellulose is useful as an excipient for a sustained release dosage form which means that it has the function to regulate the release of an active ingredient from the dosage form over an extended period of time. The term "sustained release" is used herein synonymously with the term "controlled release". Sustained release is an approach by which active ingredients such as physiologically active compounds are made available at a rate and duration designed to accomplish an intended effect. The methylcellulose is useful for forming all or part of a polymeric matrix in which the active ingredient is embedded. The polymeric matrix may additionally comprise one or more other polymers capable of providing sustained release of the active ingredient from the dosage form. The methylcellulose typically constitutes at least 50%, preferably 60-100%, more preferably 70-100%, even more preferably 80-100%, and most preferably 90-100% by weight of the polymeric matrix. When one or more other polymers are included in the polymeric matrix, they may be selected from cellulose ethers such as hydroxypropyl methylcellulose (HPMC), hydroxyethyl methylcellulose, hydroxypropyl cellulose or carboxymethyl cellulose, or they may be selected from other polysaccharides such as sodium alginate or calcium alginate. It is, however, generally preferred that the methylcellulose constitutes 100% by weight of the polymeric matrix.

The methylcellulose may be included in sustained release dosage forms, in particular dosage forms intended for oral administration of drugs or other physiologically active ingredients and release thereof into the gastrointestinal tract so as to control the absorption rate of the active ingredient to achieve a desired blood plasma profile. The combined amount of methylcellulose and active ingredient in the dosage form is preferably at least 50%, more preferably at least 70%, and most preferably at least 90% by dry weight of the dosage form, and preferably up to 100%, more preferably up to 98% and most preferably up to 95% by dry weight of the dosage form. The dosage form is designed to provide a constant or nearly constant level of the active ingredient in plasma with reduced fluctuation via a slow, continuous release of the active ingredient over an extended period of time such as a period of between 4 and 30 hours, preferably between 8 and 24 hours to release all or almost all of the active ingredient from the dosage form.

It has been found that solid sustained release dosage forms such as tablets and capsules wherein the polymer matrix is formed partially or completely from SG methylcellulose or A methylcellulose remains intact over an extended time period such as at least 4 hours, preferably at least 6 hours and under optimized conditions at least 8 hours. Without wanting to be bound by theory, it is believed that the methylcellulose is hydrated to form a strong swollen layer on the outer surface of the dosage form upon contact with an aqueous liquid at body temperature. The strong swollen layer minimizes the release of the active ingredient caused by erosion of the dosage form. Since the tablets or capsule contents do not disintegrate (i.e. do not fall apart to any significant degree), the release of the active ingredient is controlled by the slow diffusion from the swollen layer that has been formed by hydration of the methylcellulose on the outer surface of the dosage form. A strong swollen layer reduces the penetration of water into the sustained release dosage form, which delays the release of the active ingredient, particularly a water-soluble active ingredient, into an aqueous environment due to a reduced amount of water in the zone of the dosage form into which water diffuses and dissolves the active ingredient.

While the concentration of methylcellulose in the composition may vary between wide limits, it has surprisingly been found that essentially the same rate of release of the active ingredient can be achieved when a much lower amount of methylcellulose is included as all or part of the polymeric matrix. Thus, it has been found that an acceptable rate of release of the active ingredient can be achieved compared to commercial sustained release dosage forms that typically contain about 30% by weight of HPMC when the methylcellulose is included in the dosage form as the sole matrix polymer in a concentration of 0.1-10%, preferably 0.2-5.0%, more preferably 0.5-4.0%, more preferably 0.75-2.0% and still more preferably 0.8-1.5% by dry weight of the active ingredient. In one embodiment, the methylcellulose is included in the dosage form as the sole matrix polymer in a concentration of about 1% by dry weight of the active ingredient. The resulting sustained release dosage form, such as tablet or capsule, is smaller in size and therefore easier to ingest. It has furthermore been found that a satisfactory release rate may be obtained without adding any other excipients to the dosage form, though a surfactant may optionally be added during the manufacturing process as a defoaming agent.

In an embodiment, the composition comprises an additive which on ingestion reacts with gastric fluid to generate a gas such as CO₂. The developing gas is trapped in the hydrogel which, as a result, floats to the surface of the gastric contents resulting in prolonged gastric retention time. The prolonged gastric retention time improves the bioavailability of the active ingredient, increases the duration of release and improves the solubility of active ingredients that are not readily soluble in the high pH environment of the intestine. Examples of additives which generate gas in contact with gastric fluid are alkali metal or alkaline earth metal carbonates such as CaCO₃ or Na₂CO₃. The concentration of the additive may be in the range of 1-5% by weight, preferably 1.5-3% by weight, such as 2% by weight of the composition.

The present composition may suitably be prepared by providing a solution of methylcellulose in a liquid diluent, optionally adding a surfactant to the solution as a processing aid. The active ingredient in powder or crystalline form and optionally one or more solid excipients (collectively termed "solids" herein) may then be mixed with the methylcellulose solution such that the ratio of methylcellulose solution to solids is in the range of 0.1:1 to 0.85:1. The liquid diluent is preferably an aqueous liquid containing 50-100% water and may for instance be selected from purified water or water containing a surfactant acting as a defoaming aid during preparation of the composition. The weight ratio of liquid diluent to solids is preferably in the range of 0.1:1 to 0.75:1, 0.1:1 to 0.70:1, 0.1:1 to 0.65:1, 0.1:1 to 0.60:1, 0.1:1 to 0.60:1, 0.1:1 to 0.55:1, 0.1:1 to 0.50:1, 0.1:1 to 0.45:1 or 0.1:1 to 0.40:1. Addition of a surfactant helps to distribute a low level of liquid diluent homogenously and produce a smooth highly viscous semi-solid paste, possibly due to defoaming and emulsification. The surfactant may be selected from conventional defoaming agents selected from the group consisting of anionic surfactants with anionic functional groups such as sulfates, sulfonates, phosphates and carboxylates such as alkyl sulfates, e.g. ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate; cationic surfactants with cationic functional groups such as cetrimonium bromide (CTAB), cetylpyridinium chloride (CPC), benzalkonium chloride (BAC), benzethonium chloride (BZT), dimethyldioctadecylammonium chloride, dioctadecyldimethylammonium bromide (DODAB); zwitterionic surfactants such as cocamidopropyl betaine; and nonionic surfactants such as siloxane surfactants like modified polydimethylsiloxane-based defoamer, ethoxylates, fatty acid esters of glycerol, sorbitol and sucrose. The concentration of surfactant may be in the range of 0.1-1.5% by weight of the composition.

In one embodiment of the invention, the composition comprising methylcellulose admixed with the active ingredient is in the form of a dry powder. As appears from Figs. 1 and 2 appended hereto, the dried composition generally results in a slower release of the active ingredient under the conditions described in the examples below. The dry powder may be prepared by drying the mixture of the methylcellulose solution and active ingredient at a temperature of 40-100°C until the mixture has a water content of less than 10% by weight, preferably less than 5% by weight, more preferably less than 3% by weight, in particular less than 2% by weight, such as less than 1% by weight, followed by milling or grinding the mixture to granules of a desired particle size in a manner known in the art. The dry powder will typically contain granules comprising the active ingredient partially or completely encased by methylcellulose which facilitates sustained release of the active ingredient as discussed above.

In one embodiment, the invention relates to a unit dosage form comprising the present composition. The unit dosage form is intended for oral administration and may be in the form of a tablet comprising compressed granules of the dried composition. Alternatively, the unit dosage form may be in the form of a tablet or pellet prepared by extruding the semi-solid paste prepared as described above and cutting the extruded mass into pieces of an appropriate size followed by drying. The tablet may optionally comprise one or more other excipients, though preferably methylcellulose is the only excipient included in the dosage form, except that a surfactant may optionally also be included as indicated above. The unit dosage form may also be a capsule including the dried composition, preferably in the form of dry granules containing the mixture of methylcellulose and active ingredient. The unit dosage form may also be in the form of a syringe or pouch pre-filled with the wet mixture: this dosage form may more readily be administered to young children.

The unit dosage form contains one or more physiologically active ingredients, preferably one or more drugs, one or more diagnostic agents, or one or more physiologically active ingredients which are useful for cosmetic or nutritional purposes. The term "drug" denotes a compound having beneficial prophylactic and/or therapeutic properties when administered to an individual, typically a mammal, especially a human individual. The dosage form is believed to be particularly suited for administering highly dosed drugs, i.e. drugs administered in unit doses of 500 mg or more, as it is possible to provide a unit dose that includes the requisite amount of the active ingredient in a size that makes it easier to ingest. Examples of highly dosed drugs are metformin, metformin hydrochloride, acetaminophen (paracetamol) or acetylsalicylic acid. Thus, each unit dosage form may typically include 500-1000 mg of the active ingredient.

Some embodiments of the invention will now be described in detail in the following Examples.

Unless otherwise mentioned, all parts and percentages are by weight. In the Examples the following test procedures are used.

### Determination of s23/s26 of Methylcellulose

The approach to measure the ether substituents in methylcellulose is generally known. See for example the approach described in principle for Ethyl Hydroxyethyl Cellulose in Carbohydrate Research, 176 (1988) 137-144, Elsevier Science Publishers B.V., Amsterdam, DISTRIBUTION OF SUBSTITUENTS IN O-ETHYL-O-(2-HYDROXYETHYL)CELLULOSE by Bengt Lindberg, Ulf Lindquist, and Olle Stenberg.

Specifically, determination of s23/s26 was conducted as follows: 10-12 mg of the methylcellulose were dissolved in 4.0 mL of dry analytical-grade dimethyl sulfoxide (DMSO) (Merck, Darmstadt, Germany, stored over 0.3nm molecular sieve beads) at about 90 °C with stirring and then cooled to room temperature. The solution was stirred at room temperature over night to ensure complete solubilization/dissolution. The entire perethylation including the solubilization of the methylcellulose was performed using a dry nitrogen atmosphere in a 4 mL screw cap vial. After solubilization, the dissolved methylcellulose was transferred to a 22-mL screw-cap vial to begin the perethylation process. Powdered sodium hydroxide (freshly pestled, analytical grade, Merck, Darmstadt, Germany) and ethyl iodide (for synthesis, stabilized with silver, Merck-Schuchardt, Hohenbrunn, Germany) were introduced in a thirty-fold molar excess relative to the level of anhydroglucose units in the methylcellulose, and the mixture was vigorously stirred under nitrogen in the dark for three days at ambient temperature. The perethylation was repeated with addition of the threefold amount of the reagents sodium hydroxide and ethyl iodide compared to the first reagent addition, and stirring at room temperature was continued for an additional two days. Optionally, the reaction mixture could be diluted with up to 1.5 mL DMSO to ensure good mixing during the course of the reaction. Next, five mL of 5 % aqueous sodium thiosulfate solution was poured into the reaction mixture, and the mixture was then extracted three times with 4 mL of dichloromethane. The combined extracts were washed three times with 2 mL of water. The organic phase was dried with anhydrous sodium sulfate (about1 g). After filtration, the solvent was removed with a gentle stream of nitrogen, and the sample was stored at 4 °C until needed.

Hydrolysis of about 5 mg of the perethylated samples was performed under nitrogen in a 2-mL screw-cap vial with 1 mL of 90 % aqueous formic acid under stirring at 100 °C for 1 hour. The acid was removed in a stream of nitrogen at 35-40 °C and the hydrolysis was repeated with 1 mL of 2M aqueous trifluoroacetic acid for 3 hours at 120 °C in an inert nitrogen atmosphere with stirring. After completion, the acid was removed to dryness in a stream of nitrogen at ambient temperature using ca. 1 mL of toluene for co-distillation.

The residues of the hydrolysis were reduced with 0.5 mL of 0.5-M sodium borodeuteride in 2N aqueous ammonia solution (freshly prepared) for 3 hours at room temperature with stirring. The excess reagent was destroyed by dropwise addition of about 200 µL of concentrated acetic acid. The resulting solution is evaporated to dryness in a stream of nitrogen at about 35-40 °C and subsequently dried in vacuum for 15 min at room temperature. The viscous residue was dissolved in 0.5 mL of 15 % acetic acid in methanol and evaporated to dryness at room temperature. This was done five times and repeated four additional times with pure methanol. After the final evaporation, the sample was dried in vacuum overnight at room temperature.

The residue of the reduction was acetylated with 600 µL of acetic anhydride and 150 µL of pyridine for 3 hrs at 90 °C. After cooling, the sample vial was filled with toluene and evaporated to dryness in a stream of nitrogen at room temperature. The residue was dissolved in 4 mL of dichloromethane and poured into 2 mL of water and extracted with 2 mL of dichloromethane. The extraction was repeated three times. The combined extracts were washed three times with 4 mL of water and dried with anhydrous sodium sulfate. The dried dichloromethane extract was subsequently submitted to GC analysis. Depending on the sensitivity of the GC system, a further dilution of the extract could be necessary.

Gas-liquid (GLC) chromatographic analyses were performed with Agilent 6890N type of gas chromatographs (Agilent Technologies GmbH, 71034 Boeblingen, Germany) equipped with Agilent J&W capillary columns (30 m, 0.25-mm ID, 0.25-µm phase layer thickness) operated with 1.5-bar helium carrier gas. The gas chromatograph was programmed with a temperature profile that held constant at 60 °C for 1 min, heated up at a rate of 20 °C / min to 200 °C, heated further up with a rate of 4 °C / min to 250 °C, and heated further up with a rate of 20 °C / min to 310 °C where it was held constant for another 10 min. The injector temperature was set to 280 °C and the temperature of the flame ionization detector (FID) was set to 300 °C. Exactly 1µL of each sample was injected in the splitless mode at 0.5-min valve time. Data were acquired and processed with a LabSystems Atlas work station.

Quantitative monomer composition data were obtained from the peak areas measured by GLC with FID detection. Molar responses of the monomers were calculated in line with the effective carbon number (ECN) concept but modified as described in the table below. The effective carbon number (ECN) concept has been described by Ackman (R.G. Ackman, J. Gas Chromatogr., 2 (1964) 173-179 and R.F. Addison, R.G. Ackman, J. Gas Chromatogr., 6 (1968) 135-138) and applied to the quantitative analysis of partially alkylated alditol acetates by Sweet et. Al (D.P. Sweet, R.H. Shapiro, P. Albersheim, Carbohyd. Res., 40 (1975) 217-225).

### ECN increments used for ECN calculations:

| Type of carbon atom | ECN increment |
|---|---|
| hydrocarbon | 100 |
| primary alcohol | 55 |
| secondary alcohol | 45 |

In order to correct for the different molar responses of the monomers, the peak areas were multiplied by molar response factors MRFmonomer which are defined as the response relative to the 2,3,6-Me monomer. The 2,3,6-Me monomer were chosen as reference since it was present in all samples analyzed in the determination of s23 / s26.
MRFmonomer = ECN2,3,6-Me /ECNmonomer

The mol fractions of the monomers were calculated by dividing the corrected peak areas by the total corrected peak area according to the following formulas:
(1) s23 is the sum of the molar fractions of anhydroglucose units which meet the following condition [the two hydroxy groups in the 2- and 3-positions of the anhydroglucose unit are substituted with methyl groups, and the 6-position is not substituted (= 23-Me)]; and
(2) s26 is the sum of the molar fractions of anhydroglucose units which meet the following condition [the two hydroxy groups in the 2- and 6-positions of the anhydroglucose unit are substituted with methyl groups, and the 3-position is not substituted (= 26-Me)].

### Determination of the DS(methyl) a methylcellulose

The determination of the % methoxyl in methylcellulose was carried out according to the United States Pharmacopeia (USP34). The values obtained were % methoxyl. These were subsequently converted into degree of substitution (DS) for methyl substituents. Residual amounts of salt were taken into account in the conversion.

### Production of a 2 % pure aqueous solution of the methylcellulose

To obtain a 2 % aqueous solution of methylcellulose, 3 g of milled, ground, and dried methylcellulose (under consideration of the water content of the methylcellulose) were added to 147 g of tap water (temperature 20 - 25 °C) at room temperature while stirring with an overhead lab stirrer at 750 rpm with 3-wing (wing = 2 cm) blade stirrer. The solution was then cooled to about 1.5 °C. After the temperature of 1.5 °C was reached the solution was stirred for 180 min at 750 rpms. Prior to use or analysis, the solution was stirred for 15 min at 100 rpm in an ice bath.

### Determination of the viscosity of methylcellulose

The steady-shear-flow viscosity η(5 °C, 10 s⁻¹, 2 wt.% MC) of an aqueous 2-wt.% methylcellulose solution was measured at 5 °C at a shear rate of 10 s⁻¹ with an Anton Paar Physica MCR 501 rheometer and cup and bob fixtures (CC-27).

### Example 1: Release of Acetaminophen from gelatin capsules comprising SG methylcellulose

A 2% by weight aqueous solution of Methocel SGA16M methylcellulose (available from DuPont) was prepared and a modified polydimethylsiloxane-based defoamer (available from BASF under the trade name Foamstar SI2210) was added to the solution. 9.75 g of acetaminophen (abbreviated herein to APAP) was intimately mixed with 5.25 g of the solution of SGA16M methylcellulose until a white homogenous and highly viscous paste was obtained. The content of Foamstar SI2210 in the paste was 0.0885 g. The mixture was filled into a syringe and injected into gelatin capsules (size 000) which were subsequently closed and sealed. Each capsule contained about 1 g of APAP and 100 mg of SGA16M methylcellulose. The filled capsules were immediately placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 1 from which it appears that about 80% of the APAP was released from each capsule after 24 hours (shown as **-◆-** in the figure).

### Example 2: Release of Acetaminophen from dried gelatin capsules containing SG methylcellulose

A 2% by weight aqueous solution of SGA16M methylcellulose was prepared and a modified polydimethylsiloxane-based defoamer (available from BASF under the trade name Foamstar SI2210) was added to the solution. 9.75 g of APAP was intimately mixed with 5.25 g of the solution of SGA16M methylcellulose until a white homogenous and highly viscous paste was obtained. The content of Foamstar SI2210 in the paste was 0.0885 g. Gelatin capsules (size 000) were filled with the paste and subsequently closed. The mixture was carefully dried overnight at room temperature. Each capsule contained about 1 g of APAP and 100 mg SGA16M methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the dried capsules is shown in Fig. 1 from which it appears that about 70% of the drug was released after 24 hours (shown as **-■-** in the figure).

### Example 3: Release of Acetaminophen from gelatin capsules comprising SG methylcellulose

A 2% by weight aqueous solution of Methocel SGA7C methylcellulose (available from DuPont) was prepared and a modified polydimethylsiloxane-based defoamer (available from BASF under the trade name Foamstar SI2210) was added to the solution. 9.75 g of acetaminophen (abbreviated herein to APAP) was intimately mixed with 5.25g of the solution of SGA7C methylcellulose until a white homogenous and highly viscous paste was obtained. The content of Foamstar SI2210 in the paste was 0.0885 g. The mixture was filled into a syringe and injected into gelatin capsules (size 000) which were subsequently closed. Each capsule contained about 1 g of APAP and 100 mg of SGA7C methylcellulose. The filled capsules were immediately placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 2 from which it appears that about 85% of the APAP was released from each capsule within 6 hours and 90% of the APAP was released from each capsule after 24 hours (shown as **-◆-** in the figure).

### Example 4: Release of Acetaminophen from dried gelatin capsules containing SG methylcellulose

A 2% by weight aqueous solution of SGA7C methylcellulose was prepared and a modified polydimethylsiloxane-based defoamer (available from BASF under the trade name Foamstar SI2210) was added to the solution. 9.75g of APAP was intimately mixed with 5.25 g of the solution of SGA7C methylcellulose until a white homogenous and highly viscous paste was obtained. The content of Foamstar SI2210 in the paste was 0.0885 g. Gelatin capsules (size 000) were filled with about 1 g of the mixture and subsequently closed. The mixture was dried overnight at room temperature. Each capsule contained about 1 g of APAP and 100 mg SG methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 2 from which it appears that about 90% of the drug was released after 24 hours (shown as -■- in the figure). It further appears that the rate of release from the dried capsules is slower than the rate of release from wet capsules.

### Example 5: Release of acetaminophen from dried gelatin capsules containing SG methylcellulose (according to the invention) and A methylcellulose (not according to the invention)

2% by weight aqueous solutions of SGA16M methylcellulose and A4M methylcellulose were prepared and a modified polydimethylsiloxane-based defoamer (available from BASF under the tradename Foamstar SI 2210) was added to each solution. 9.75 g of APAP was intimately mixed with 5.25 g of the respective solutions of SGA16M methylcellulose and A4M methylcellulose until a white homogenous and highly viscous paste was obtained. The content of Foamstar SI2210 in each paste was 0.0885 g. Gelatin capsules (size 000) were filled with about 1 g of each paste and subsequently closed. The capsules were then dried carefully overnight at 50°C. Each capsule contained about 1 g of APAP and 100 mg SGA16M methylcellulose or A4M methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the capsules is shown in Fig. 3 from which it appears that about 70% of the drug was released after 24 hours from the capsules containing SGA16M methylcellulose as the sustained release polymeric matrix, whereas about 90% of the drug was released after 6 hours from the capsules containing A4M methylcellulose. A polymeric matrix composed of SGA16M methylcellulose therefore appears to be particularly suitable for oral dosage forms for once daily administration.

### Example 6: Release of acetaminophen from dried gelatin capsules containing SG methylcellulose and CaCO₃

A 2% by weight aqueous solution of SGA16M methylcellulose was prepared and 2% by weight CaCO₃ was added to the solution. 9.75 g of APAP was intimately mixed with 5.25 g of the solution of SGA16M methylcellulose and CaCO₃ until a white homogenous and highly viscous paste was obtained. Gelatin capsules (size 000) were filled with the paste and subsequently closed. The capsules were carefully dried overnight at 50°C and for two days at room temperature, respectively. Each capsule contained about 1 g of APAP and 100 mg SGA16M methylcellulose. The dried capsules were placed in 900 ml of 0.1N HCl pH 1.1 at 37°C and shaken at 150 rpm for 22 hours. Drug release was measured at a wavelength of 243 nm with a path length of 0.1 mm.

The release of APAP from the dried capsules is shown in Fig. 4 from which it appears that about 65% of the drug was released after 24 hours from the capsule dried for 2 days at room temperature (shown as **-■-** in the figure), and that about 60% of the drug was released after 24 hours from the capsule dried overnight at 50°C (shown as - ◆ - in the figure).

It was observed that after immersion in the simulated gastric fluid, the capsules containing CaCO₃ floated at the surface, while corresponding capsules that did not contain CaCO₃ sank to the bottom of the test liquid.

## Claims

1. A sustained release composition for oral administration comprising a physiologically active ingredient mixed with a methylcellulose, wherein
the methylcellulose has anhydroglucose units joined by 1-4 linkages and wherein hydroxy groups of anhydroglucose units are substituted with methyl groups such that the s23/s26 is between 0.27 and 0.33,
wherein s23 is the molar fraction of anhydroglucose units wherein only the two hydroxy groups in the 2- and 3-positions of the anhydroglucose unit are substituted with methyl groups and
wherein s26 is the molar fraction of anhydroglucose units wherein only the two hydroxy groups in the 2- and 6-positions of the anhydroglucose unit are substituted with methyl groups, and
wherein the concentration of methylcellulose is from 0.1% to 10% by dry weight of the active ingredient.

2. The composition of claim 1, wherein the concentration of methylcellulose is 0.2-5%, preferably 0.5-4%, more preferably 0.75-2% and still more preferably 0.8-1.5%, by dry weight of the active ingredient.

3. The composition of claim 1, wherein the concentration of methylcellulose is about 1% by dry weight of the active ingredient.

4. The composition of any one of claims 1 to 3 comprising a methylcellulose wherein hydroxy groups of anhydroglucose units are substituted with methyl groups such that s23/s26 is between 0.27 and 0.30.

5. The composition of any one of claims 1 to 4 wherein the methylcellulose has a DS(methyl) of from 1.55 to 2.25.

6. The composition of any one of claims 1 to 5, wherein the methylcellulose has a viscosity of from 2.4 to 10000 mPa•s, measured as 2 wt. % aqueous solution at 5 °C at a shear rate of 10 s⁻¹.

7. The composition of any one of claims 1 to 6, wherein the methylcellulose comprises at least 50%, preferably 60-100%, by weight of a polymeric matrix in which particles of the active ingredient are embedded.

8. The composition of any one of claims 1 to 7 further comprising a surfactant.

9. The composition of any one of claims 1 to 8, wherein the concentration of the surfactant is in the range of 0.1-1.5% by weight of the composition.

10. The composition according to any one of claims 1 to 9 further comprising an additive capable of reacting with gastric fluid to generate a gas.

11. The composition according to claim 10, wherein the additive is selected from alkali metal and alkaline earth metal carbonates such as CaCO₃ or Na₂CO₃.

12. The composition of any one of claims 1 to 11 in the form of a dry powder.

13. A unit dosage form comprising a composition according to any one of claims 1 to12.

14. The unit dosage form of claim 13 comprising 500-1000 mg of the active ingredient.

15. The unit dosage form of claim 14, wherein the active ingredient is selected from the group consisting of metformin, metformin hydrochloride, acetaminophen and acetylsalicylic acid.

## Patentansprüche

1. Eine Retardzusammensetzung zur oralen Verabreichung umfassend einen physiologisch aktiven Wirkstoff gemischt mit einer Methylcellulose, wobei die Methylcellulose Anhydroglucoseeinheiten verknüpft mit 1 bis 4 Verknüpfungen aufweist und wobei die Hydroxygruppen der Anhydroglucoseeinheiten in einer solchen Weise mit Methylgruppen substituiert sind, dass das s23/s26-Verhältnis zwischen 0,27 und 0,33 beträgt,
wobei s23 die molare Fraktion von Anhydroglucoseeinheiten ist, in denen nur die zwei Hydroxygruppen in den 2- und 3-Positionen der Anhydroglucoseeinheiten mit Methylgruppen substituiert sind und
wobei s26 die molare Fraktion von Anhydroglucoseeinheiten ist, in denen nur die zwei Hydroxygruppen in den 2- und 6-Positionen der Anhydroglucoseeinheiten mit Methylgruppen substituiert sind und
wobei die Konzentration der Methylcellulose von 0,1% bis 10% des Trockengewichts des Wirkstoffs beträgt.

2. Die Zusammensetzung gemäß Anspruch 1, wobei die Konzentration der Methylcellulose 0,2 bis 5%, vorzugsweise 0,5 bis 4%, bevorzugter 0,75 bis 2% und noch bevorzugter 0,8 bis 1,5%, bezogen auf das Trockengewicht des Wirkstoffs beträgt.

3. Die Zusammensetzung gemäß Anspruch 1, wobei die Konzentration der Methylcellulose etwa 1% des Trockengewichts des Wirkstoffs beträgt.

4. Die Zusammensetzung gemäß irgendeinem der Ansprüche 1 bis 3 umfassend eine Methylcellulose, in welcher Hydroxygruppen der Anhydroglucoseeinheiten mit Methylgruppen in einer solchen Weise substituiert sind, dass das s23/s26-Verhältnis zwischen 0,27 und 0,30 beträgt.

5. Die Zusammensetzung gemäß irgendeinem der Ansprüche 1 bis 4, wobei die Methylcellulose einen DS(Methyl) von 1,55 bis 2,25 aufweist.

6. Die Zusammensetzung gemäß irgendeinem der Ansprüche 1 bis 5, wobei die Methylcellulose eine Viskosität von 2,4 bis 10.000 mPa·s, gemessen als 2 gew.-%-ige wässrige Lösung bei 5 °C und einer Scherrate von 10 s⁻¹, aufweist.

7. Die Zusammensetzung gemäß irgendeinem der Ansprüche 1 bis 6, wobei die Methylcellulose mindestens 50 Gew.-%, vorzugsweise 60 bis 100 Gew.-% einer polymeren Matrix umfasst, in welche Teilchen des Wirkstoffs eingebettet sind.

8. Die Zusammensetzung gemäß irgendeinem der Ansprüche 1 bis 7 des Weiteren umfassend ein oberflächenaktives Mittel.

9. Die Zusammensetzung gemäß irgendeinem der Ansprüche 1 bis 8, wobei die Konzentration des oberflächenaktiven Mittels im Bereich von 0,1 bis 1,5 Gew.-% der Zusammensetzung liegt.

10. Die Zusammensetzung gemäß irgendeinem der Ansprüche 1 bis 9 des Weiteren umfassend ein Additiv, welches mit Magensaft reagieren kann, um ein Gas zu erzeugen.

11. Die Zusammensetzung gemäß Anspruch 10, wobei das Additiv aus Alkalimetall- und Erdalkalimetallcarbonaten, wie beispielsweise CaCO₃ oder Na₂CO₃, ausgewählt ist.

12. Die Zusammensetzung gemäß irgendeinem der Ansprüche 1 bis 11 in Form eines trockenen Pulvers.

13. Eine Einzeldosisform umfassend eine Zusammensetzung gemäß irgendeinem der Ansprüche 1 bis 12.

14. Die Einzeldosisform gemäß Anspruch 13 umfassend 500 bis 1.000 mg des Wirkstoffs.

15. Die Einzeldosisform gemäß Anspruch 14, wobei der Wirkstoff aus der Gruppe bestehend aus Metformin, Metforminhydrochlorid, Acetaminophen und Acetylsalicylsäure ausgewählt ist.

## Revendications

1. Composition à libération prolongée pour administration par voie orale, comprenant un ingrédient physiologiquement actif mélangé avec une méthyl-cellulose,
laquelle méthyl-cellulose présente des motifs d'anhydro-glucose reliés par des liaisons 1-4 et dans laquelle les groupes hydroxyle des motifs d'anhydro-glucose sont remplacés par des groupes méthyle de telle sorte que le rapport s23/s26 vaut entre 0,27 et 0,33,
étant entendu que s23 désigne la fraction molaire des motifs d'anhydro-glucose chez lesquels seuls les deux groupes hydroxyle placés en les positions 2 et 3 du motif d'anhydro-glucose sont remplacés par des groupes méthyle,
et que s26 désigne la fraction molaire des motifs d'anhydro-glucose chez lesquels seuls les deux groupes hydroxyle placés en les positions 2 et 6 du motif d'anhydro-glucose sont remplacés par des groupes méthyle,
et dans laquelle composition la proportion de méthyl-cellulose vaut de 0,1 % à 10 %, par rapport au poids à sec de l'ingrédient actif.

2. Composition conforme à la revendication 1, dans laquelle la proportion de méthyl-cellulose vaut de 0,2 à 5 %, de préférence de 0,5 à 4 %, mieux encore de 0,75 à 2 % et surtout de 0,8 à 1,5 %, par rapport au poids à sec de l'ingrédient actif.

3. Composition conforme à la revendication 1, dans laquelle la proportion de méthyl-cellulose vaut environ 1 %, par rapport au poids à sec de l'ingrédient actif.

4. Composition conforme à l'une des revendications 1 à 3, qui comprend une méthyl-cellulose dans laquelle les groupes hydroxyle des motifs d'anhydro-glucose sont remplacés par des groupes méthyle de telle sorte que le rapport s23/s26 vaut entre 0,27 et 0,30.

5. Composition conforme à l'une des revendications 1 à 4, dans laquelle la méthyl-cellulose présente un degré DS(méthyle) valant de 1,55 à 2,25.

6. Composition conforme à l'une des revendications 1 à 5, dans laquelle la méthyl-cellulose présente une viscosité, mesurée en solution aqueuse à 2 % en poids, à 5 °C et sous un gradient de vitesse de cisaillement de 10 s⁻¹, valant de 2,4 à 10 000 mPa.s.

7. Composition conforme à l'une des revendications 1 à 6, dans laquelle la méthyl-cellulose comprend au moins 50 % et de préférence de 60 à 100 % en poids d'une matrice de polymère au sein de laquelle sont incorporées des particules de l'ingrédient actif.

8. Composition conforme à l'une des revendications 1 à 7, qui comprend en outre un tensioactif.

9. Composition conforme à l'une des revendications 1 à 8, dans laquelle la proportion de tensioactif se situe dans l'intervalle allant de 0,1 à 1,5 % par rapport au poids de la composition.

10. Composition conforme à l'une des revendications 1 à 9, qui comprend en outre un adjuvant capable de réagir avec le suc gastrique pour produire un gaz.

11. Composition conforme à la revendication 10, dans laquelle l'adjuvant est choisi parmi les carbonates de métal alcalin ou de métal alcalino-terreux, comme CaCO₃ ou Na₂CO₃.

12. Composition conforme à l'une des revendications 1 à 11, qui se présente sous forme de poudre sèche.

13. Forme posologique unitaire comprenant une composition conforme à l'une des revendications 1 à 12.

14. Forme posologique unitaire conforme à la revendication 13, qui comprend de 500 à 1000 mg de l'ingrédient actif

15. Forme posologique unitaire conforme à la revendication 14, dans laquelle l'ingrédient actif est choisi dans l'ensemble constitué par la metformine, le chlorhydrate de metformine, l'acétaminophène et l'acide acétyl-salicylique.
